# EUROPEAN PATENT SPECIFICATION

(11) **EP 4 308 537 B1**
(45) Date of publication and mention of the grant of the patent: **19.02.2025**
(21) Application number: 22715568.6
(22) Date of filing: 15.03.2022
(51) Int. Cl.: C07C 51/02, C07C 51/09, C07C 51/41, C07C 51/47, C07C 51/48, C07C 55/18

(54) **RECOVERY OF DICARBOXYLIC ACIDS FROM TRIGLYCERIDES**
RÜCKGEWINNUNG VON DICARBONSÄUREN AUS TRIGLYCERIDEN
RÉCUPÉRATION D'ACIDES DICARBOXYLIQUES À PARTIR DE TRIGLYCÉRIDES

(30) Priority: 17.03.2021 IT 202100006410
(43) Date of publication of application: 24.01.2024
(73) Proprietor: Novamont S.p.A., 28100 Novara (IT)
(72) Inventor: CAPUZZI, Luigi, 28100 Novara (IT); DIGIOIA, Francesca, 28010 Barengo (NO) (IT); QUATRALE, Lorenzo, 28100 Novara (IT)
(74) Representative: Zanoli, Enrico
(86) International application number: PCT/EP2022/056692
(87) International publication number: WO 2022/194863

(56) References cited:
- WO-A1-2017/211766

## Description

The project that led to the invention was funded by the Bio Based Industries Joint Undertaking Public-Private Partnership under the European Union's Horizon research and innovation programme 2020, under Grant Agreement No. 745766.

The present invention relates to a process for recovering dicarboxylic acids from mixtures of dicarboxylic acid triglycerides obtained by the oxidative cleavage of vegetable oils.

The need to use raw materials from renewable sources to ensure the environmental sustainability of industrial production is now strongly felt in the production of consumer goods, especially in the plastics sector. Bioplastics are therefore being developed in this sector, where traditional petroleum-derived monomers are being replaced by raw materials from renewable sources.

One example of monomers of natural origin is dicarboxylic acids obtained from the processes of oxidative cleavage of vegetable oils and subsequent purification, such as the processes described in international patent applications WO2008 / 138892A1, WO 2011/080296 A1 and WO 2017/ 211766A1.

In these processes, the product from oxidative cleavage consists of mixtures of triglycerides containing saturated dicarboxylic acids and monocarboxylic acids, which are typically removed by distillation.

The acid triglyceride mixtures obtained by oxidative cleavage then undergo a hydrolysis reaction to separate the dicarboxylic acids from the glyceride skeleton. To limit the use of chemicals, the hydrolysis reaction can be carried out with water at high pressures and temperatures; alternatively, the hydrolysis can be catalysed by bases or acids, for example strongly acidic ion exchange resins, or catalysed by enzymes such as lipases.

With the same aim of limiting environmental impact and limiting the use of organic solvents, the product of the hydrolysis reaction with water at high pressures and temperatures in the above-mentioned processes is subsequently purified by exploiting the solubility of the glycerol and diacids in water.

However, the organic phase remaining after hydrolysis is still rich in mono- and dicarboxylic acids. In order to increase recovery yields the process described in application WO 2017/211766 A1 envisages, for example, further subjecting this organic phase (see step c) to evaporation and/or distillation, thereby separating the dicarboxylic acids still present, on the basis of their respective boiling points, from the monocarboxylic acids with different chain lengths of carbon atoms and from further components of the organic phase (for example non-hydrolysed or partly hydrolysed glycerides, partial oxidation products resulting from previous process steps). However, recovery operations involving high temperatures favour the formation of secondary condensation products that acquire high stability to hydrolysis and thermal oxidation, thus leading to a loss of product.

It is therefore necessary to identify a process for recovering dicarboxylic acids from complex triglyceride mixtures obtained by the oxidative cleavage of vegetable oils that is not subject to the above-mentioned disadvantages and that allows for increased utilisation of the renewable resources used.

Of the known methods of hydrolysis, basic hydrolysis or saponification is particularly efficient in terms of yield and is therefore used, for example, in the preparation of samples for the analytical characterisation of complex products such as triglyceride oxidation products. However aqueous phase separation of dicarboxylic acids obtained by acidification of the hydrolysis product is limited, probably because of the presence of monocarboxylic acids and other residues forming the organic phase in the hydrolysis product; consequently, the recovery of dicarboxylic acids is insufficient.

In this respect, it has been surprisingly observed that, after subjecting triglycerides of dicarboxylic acids to a basic hydrolysis reaction, it is possible to increase the recovery of dicarboxylic acids by performing a two-stage acidification, and in particular by separating the aqueous phase from the organic phase after partial acidification of the hydrolysis mixture (to a neutral or weakly acidic pH, for example 4-6.5, depending on the composition of the mixture), and subsequently acidifying only the separated aqueous phase to a strongly acidic pH. Conversely, direct acidification of the mixture generated following the basic hydrolysis of triglycerides of dicarboxylic acid up to strongly acidic pH leads to their breakdown to such an extent that their recovery is impaired.

It is therefore an object of the present invention to provide a process for obtaining dicarboxylic acids from a mixture of triglycerides of dicarboxylic acids comprising the steps of:
a) hydrolysing said mixture of triglycerides of dicarboxylic acids in the presence of a basic aqueous solution, obtaining an emulsion of the hydrolysis product;
b) acidifying the product from step a) until the emulsion separates, preferably to a pH of between 3.5 and 7, depending on the composition of the hydrolysis product,
c) separating an aqueous phase containing dicarboxylic acids in partly dissociated form and glycerol from the product from step b),
d) acidifying the aqueous phase separated in step c) to a pH of 3 or below, thus obtaining dicarboxylic acids.

The aqueous phase separated in step d) is optionally cooled to promote crystallisation of the dicarboxylic acid.

The mixture of triglycerides of dicarboxylic acids suitable for use as starting material in this process is a mixture comprising one or more triglycerides which are the same or differ from each other and which contain at least one acyl group of a dicarboxylic acid.

Suitable dicarboxylic acids are aliphatic diacids, preferably of the alpha-omega type, for example chosen from oxalic acid, malonic acid, succinic acid, glutaric acid, adipic acid, pimelic acid, suberic acid, azelaic acid, sebacic acid, undecandicarboxylic acid, dodecandicarboxylic acid, brassylic acid, tetradecandicarboxylic acid, pentadecandicarboxylic acid. Advantageously, said dicarboxylic acids have a C6-C24 chain and preferably belong to the group comprising suberic acid, azelaic acid, sebacic acid, undecandicarboxylic acid, brassylic acid and mixtures thereof.

In addition to the aforementioned acyl groups of dicarboxylic acids, said triglycerides typically contain one or more acyl groups of the same or different monocarboxylic acids. Said monocarboxylic acids are aliphatic monoacids and may be saturated or unsaturated, substituted or unsubstituted; they typically have a chain length of C6-C24, and most commonly C9-C24. Examples of unsubstituted monocarboxylic acids are palmitic, stearic, oleic, arachic, behenic and lignoceric acids.

Examples of substituted monocarboxylic acids are long-chain monocarboxylic acids with one or more ketone groups or hydroxyl groups in a non-terminal position, such as C12-C24 carboxylic acids containing at least one ketone group or C12-C24 hydroxy acids containing at least one secondary hydroxyl group. Specific examples of substituted monocarboxylic acids that may be present are 9-hydroxystearic acid, 9-ketostearic acid, 10-ketostearic acid and 10-hydroxystearic acid.

These substituted monocarboxylic acids may contain two adjacent hydroxyl groups, such as dihydroxypalmitic, dihydroxystearic, dihydroxyoleic, dihydroxyarachic and dihydroxybehenic acids, or a hydroxyl group adjacent to a ketone group.

The mixture containing triglycerides of carboxylic acids with more than one acid functional group used as starting material in this process also optionally contains one or more free monocarboxylic and/or dicarboxylic acids.

Examples of monocarboxylic acids that may be free in mixtures are saturated or unsaturated, linear or branched, substituted or unsubstituted aliphatic monoacids of chain length C2 to C24. Examples of dicarboxylic acids that may be found free in mixtures correspond to those listed above as possible acyl substituents of triglycerides.

The mixture of dicarboxylic acid triglycerides undergoing the process according to the invention may further contain mono-, di- and triglycerides containing one or more of the following groups formed from C₆-C₂₄ dicarboxylic acid residues, C₄-C₂₄ monocarboxylic acid residues and C₄-C₂₄ alkyls. It may also contain dimer and trimer esters of glycerides containing C₆-C₂₄ dicarboxylic acid residues.

Said starting mixture preferably has an acid number of between 50 and 300 mg KOH/g. The acid number is defined as the amount of KOH, expressed in mg, used to neutralise the acidity of 1 g of substance. The determination is carried out according to ASTM D974-07 in the presence of phenolphthalein.

The Saponification Number, defined as the amount of KOH, expressed in mg, consumed in the saponification of 1 gram of substance, is preferably between 100 and 600 mg KOH/g. The Saponification Number is determined by titration of the residual KOH with HCl, in the presence of phenolphthalein, after reflux saponification for 60 minutes.

Said triglyceride mixture of starting dicarboxylic acids can advantageously be obtained, for example, from unsaturated triglycerides present in vegetable oils or animal fats using known techniques.

One example is oxidative cleavage reactions of the double bonds present in the acyl groups of said unsaturated triglycerides. These reactions may be carried out through the use of one or more oxidising agents such as inorganic and organic peroxides, peracids, nitric acid, permanganates, periodates, O₂, O₃, or mixtures of gases containing them.

In particular, mixtures of triglycerides obtained by processes of the oxidative cleavage of unsaturated triglycerides in which peroxides, such as hydrogen peroxide, and O₂ or mixtures containing O₂ are used as starting material for the present process. Examples are the processes described in WO 2008/138892, WO 2011/080296 or WO 2013/079849 A1.

According to one aspect of the invention, the triglyceride mixture of dicarboxylic acids subjected to the process according to the invention is preferably obtained as an oxidative cleavage product of vegetable oils (or mixtures of vegetable oils) containing unsaturated carboxylic acids, after separation of the monocarboxylic acids generated therefrom.

By "vegetable oils" are meant either the unmodified pressed product or an oil that has undergone chemical or physical-chemical modifications such as purification treatments or enzyme enrichment. Examples of vegetable oils are: soybean oil, olive oil, castor oil, sunflower oil, peanut oil, corn oil, palm oil, jatropha oil, cuphea oil, oils from Brassicaceae such as *Crambe abyssinica, Brassica carinata, Brassica napus* (rapeseed), Lesquerella, and other oils having a high monounsaturated acids content.

Sunflower oil and oils from Brassicaceae are particularly preferred.

Triglyceride mixtures obtained from the oxidation of sunflower oil and in particular high oleic sunflower oil (HOSO) are of particular interest as starting mixtures.

One example of a starting mixture suitable for the process according to the invention is the mixture of triglycerides obtained according to the process described in patent application WO 2008/138892 after separation of the monocarboxylic acids. Another example is the mixture of triglycerides containing carboxylic acids with more than one acid functional group obtained during the continuous oxidative cleavage process described in patent application WO 2011/080296. Particularly preferred is the use of the mixture of triglycerides obtained at the end of step c) (i.e. separation of saturated monocarboxylic acids) in said process.

According to a preferred aspect of the present invention, the starting mixture of triglycerides containing dicarboxylic acids comprises one or more of the following oligomer structures:

H-[O-C(O)-R1-C(O)-O-CH2-CH(OR2)-CH2]n-O-R3

wherein
R1 is selected from C2-C22 alkylenes,
R2 is selected from one or more of the following groups formed by C6-C24 dicarboxylic acid residues and C6-C24 monocarboxylic acid residues,
R3 is selected from one or more of the following groups formed by H, C6-C24 dicarboxylic acid residues and C6-C24 monocarboxylic acid residues,
n is an integer greater than or equal to 2.

With reference to the above structure, R1 is preferably a C6-C11 alkylene, C6, C7 and/or C11 alkylenes being particularly preferred. The two or more R1 in the structure may be different from each other.

R2 represents C6-C24 dicarboxylic acid residues or C6-C24 monocarboxylic acid residues or a mixture thereof. The two or more R2s in the structure may be different from each other.

R3 represents C6-C24 dicarboxylic acid residues or C6-C24 monocarboxylic acid residues.

Typically, such oligomer structures are dimer or trimer esters of triglycerides having a number of repetitive units (n) of 2 or 3. Particularly preferred are dimer and trimer esters of triglycerides containing C6-C24 dicarboxylic acid residues.

According to this aspect, the Saponification Number of the starting dicarboxylic acid triglyceride mixture, understood as the amount of KOH, expressed in mg, consumed in the saponification of 1 gram of substance, is preferably between 100 and 450 mg KOH/g. The Saponification Number is determined by titration of the residual KOH with HCl, in the presence of phenolphthalein, after reflux saponification for 60 minutes.

A mixture with such characteristics is for example obtained as a residue from the purification step in the process described in patent application WO 2017/211766 A1, and in particular obtained as a residue after evaporation and/or distillation in step c) of said process.

Thus, in a preferred embodiment, the process according to the invention comprises, prior to step a), the preliminary steps of:
(i) hydrolysing said mixture of triglycerides of dicarboxylic acids in the absence of a base, and
(ii) separating a first aqueous phase, comprising glycerol, from a first organic phase containing the remaining hydrolysis product from step (i),
(iii) evaporating and/or distilling said first organic phase to remove the free dicarboxylic and monocarboxylic acids contained therein,
obtaining an evaporation and/or distillation residue comprising a mixture of unhydrolysed or partially hydrolysed dicarboxylic acid glycerides.

Said hydrolysis step (i) can be performed using different techniques, for example with water alone, with strongly acidic ion exchange resins or by catalysing the reaction with enzymes.

In the case of hydrolysis with water, the reaction preferably takes place at temperatures between 150 and 350°C, more preferably between 180 and 320°C, and at pressures typically between 10 and 200 bar, with or without the addition of a catalyst. The weight ratio of water to organic phase is preferably between 1:2 and 5:1, for example between 1:1 and 5:1.

Hydrolysis with strongly acidic ion exchange resins is carried out at a temperature of 100-120°C, for example. Examples of suitable resins are Amberlyst^{®} and Amberlite^{®} (both produced by Rohm and Haas Co.).

In the case of reactions catalysed by enzymes (lipases), lipases can be advantageously selected from the group including: *Candida cylindracea, Candida antartica,* Pseudomonas sp., porcine pancreatic lipase, *Candida rugosa, Geotrichum candidum, Aspergillus niger, Mucor mietei, Rhizopus arrhizus, Rhizopus delemar, Rhizopus niveus, Chromobacterium viscosum, Thermomyces lanuginosus, Penicillum cyclopium.* Hydrolysis step (i) can be carried out by mixing an appropriate amount of dicarboxylic acids, glycerol and glycerol partial esters with the starting triglycerides, with advantages in terms of yield and process economy, as described in European patent application 17731083.6.

In optional step (ii), a first aqueous phase, containing glycerol together with varying amounts of carboxylic acids present in the hydrolysis product from step (i), which may be soluble in water, is separated from the remainder of the hydrolysis product in the organic phase. This operation is carried out according to practices known to those skilled in the art, for example by settling or centrifuging.

In order to facilitate separation of the aqueous phase from the organic phase, step (ii) may also comprise one or more operations of degassing, washing with water and/or addition of appropriate quantities of water-immiscible organic solvents.

Examples of solvents suitable for facilitating separation of the aqueous phase from the organic phase are hydrocarbons such as hexane, octane, nonane or mixtures thereof.

The operation of separating the two phases can be carried out once or more than once, if necessary, by adding fresh water and carrying out one or more successive washes of the separated organic phase, for example in counterflow.

The separated organic phase essentially contains saturated carboxylic acids with one or more acid functional groups (i.e. monocarboxylic and dicarboxylic acids) which may be substituted and which may have been released as a result of the hydrolysis reaction, triglycerides and their oligomers which have not been hydrolysed or which result from incomplete hydrolysis of the initial mixture.

In optional step (iii) this organic phase is subjected to one or more evaporation and/or distillation operations, for example chosen from evaporation in thin film evaporators, evaporation in falling film evaporators, steam distillation and molecular distillation.

These operations make it possible to separate any residual water and organic solvent and free carboxylic acids from the starting mixture comprising triglycerides of dicarboxylic acids.

In step a) of the process according to the present invention, the starting mixture of triglycerides of dicarboxylic acids is subjected to hydrolysis in the presence of an aqueous basic solution.

The term "hydrolysis" is understood here to mean the separation of a composition containing one or more glycerol esters, such as vegetable oils, mono-, di- or triglycerides of carboxylic acids, into their components (glycerol and carboxylic acids) by reacting the starting material with water.

The amount of water used in the hydrolysis reaction depends on the weight of the starting material. One embodiment of the invention uses a minimum of three moles of water for each mole of the starting material. In a preferred embodiment, the weight ratio of water to starting material is between 1:1 and 5:1, preferably with at least 4 g of water per 1 g of starting material. The hydrolysis reaction according to the invention is conducted in the presence of bases. Strong bases chosen, for example, from sodium hydroxide, potassium hydroxide, calcium hydroxide, barium hydroxide, lithium hydroxide, magnesium hydroxide are preferred. Soluble bases having the hydroxide group are preferred, sodium hydroxide being particularly preferred as it facilitates subsequent separation operations.

Aqueous bases are advantageously used at concentrations of less than 30% by weight, preferably less than 20% and even more preferably less than 10% by weight.

The hydrolysis reaction may be performed under controlled temperature and pressure conditions as indicated above for optional step (i). A preferred temperature range is from about 50°C to about 350°C; a more preferred temperature range is from about 80°C to about 150°C. Particularly preferred conditions for hydrolysis are a temperature of about 100°C at atmospheric pressure (1bar).

The hydrolysis reaction may be performed as a batch, continuous or semi-continuous method depending on the user's requirements.

The hydrolysis reaction in discontinuous or batch mode may be performed in a time interval from about 0 hours to about 6 hours. A preferred time interval for discontinuous hydrolysis is from about 2 hours to about 4 hours. A more preferred time for discontinuous hydrolysis is about 3 hours. However, semi-continuous and continuous methods allow for uninterrupted processing because water and starting material are continuously introduced into the reaction, achieving the desired degree of hydrolysis based on adjustment of the reaction parameters, including the contact time between the aqueous and organic phases.

The hydrolysis reaction is preferably carried out under conditions favouring an adequate exchange surface between the aqueous phase and the organic phase being hydrolysed, for example by operating with agitation by mechanical means or by countercurrent flow to increase the efficiency of the reaction.

After the reaction, the hydrolysis product is present in the form of an emulsion in which the organic phase typically constitutes the discontinuous phase and is dispersed in the aqueous phase, which constitutes the continuous phase. The salts of carboxylic acids formed during the hydrolysis reaction act as emulsifiers and contribute to the stability of the dispersion.

In step b) of the process according to the invention, the hydrolysis product is advantageously brought to a temperature below 95°C, preferably between 85°C and 50°C, for example a temperature around 80°C, and then partly acidified by the addition of a mineral acid.

This mineral acid is advantageously chosen from the group consisting of sulfuric acid, hydrochloric acid, nitric acid, phosphoric acid, hydrobromic acid, hydroiodic acid and perchloric acid and the like, for example in the form of an aqueous solution.

The acid is preferably added gradually and in such a quantity as to cause a reduction in the stability of the emulsion, consequent coalescence of the dispersed phase and "breaking" of the emulsion with the manifest formation of two distinct phases.

The pH reached in step b) may vary depending on the composition of the hydrolysis product and is the pH at which breaking of the emulsion takes place; typically, the separation between the phases takes place at a pH of about 3.5 to 7, more preferably 4 to 6.5, with reference to the standard temperature of 25°C.

Under these conditions, the carboxylic acids, which are in a completely dissociated form as salts in the hydrolysis product, begin to pass into an acidic form; in particular, the dicarboxylates are partly protonated.

By way of example, according to one aspect of the invention in which the starting triglyceride mixture has a saponification number of more than 350 mg KOH/g and a total dicarboxylic acids content (in both free and bound forms) of more than 25% by weight and/or a ratio of dicarboxylic to monocarboxylic acids of more than 2:1 by weight (in both free and bound forms), phase separation is achieved at a pH of 3.5 to 5.5.

According to another aspect of the invention in which the starting triglyceride mixture has a saponification number of less than 350 mg KOH/g and a total dicarboxylic acid content of less than 25% by weight (in both free and bound forms) and/or a ratio of dicarboxylic to monocarboxylic acids of less than 2:1 by weight (in both free and bound forms), phase separation is achieved at a pH of from to 5.5 to 6.7.

Step c) of this process comprises at least one liquid/liquid separation operation to separate the aqueous phase from the organic phase. This operation is carried out according to techniques known to those skilled in the art, and may for example be chosen from centrifuging, settling, solvent extraction and combinations thereof.

Said separation is advantageously performed at a temperature which favours dissolution of the dicarboxylic acid in water. A temperature of 50°C or more, preferably 70°C or more, even more preferably 80°C or more is suitable, for example. In the case where the starting triglycerides comprise mainly azelaic acid, the separation is typically carried out by settling, preferably by bringing the hydrolysis product to temperatures between 60 and 90°C and to a pressure close to atmospheric pressure (about 1 bar).

The organic phase separated at the end of step c) contains mainly saturated dicarboxylic acids and monocarboxylic acids released as a result of the hydrolysis reaction and a hydrolysis residue consisting of partial glycerides (monoglycerides and/or diglycerides) and/or unsaponifiable products.

The aqueous phase separated at the end of step c) contains mainly glycerol and dicarboxylic acids in a partly dissociated form. The Applicant has observed that, operating on the hydrolysis product of the mixtures of acid triglycerides described above, when separation of the aqueous phase is carried out at the pH corresponding to the "break" point of the emulsion, breakdown of said dicarboxylic acids in water is surprisingly favoured. In fact, an increase in the final recovered yield of dicarboxylic acids of even more than 50%, more than 60%, more than 70% and advantageously more than 80% with respect to the recovered yield in the separation carried out by acidifying directly at a strongly acid pH is obtained.

In step d) of the process, said aqueous phase separated in step c) is completely acidified to obtain the dicarboxylic acids in acid form. Said aqueous phase is acidified until the carboxylic acids are completely protonated; this condition typically occurs at a pH of 3 or below, preferably below 2, more preferably at pH 1, with reference to the standard temperature of 25°C, yielding dicarboxylic acids.

According to one aspect of the process, in step d) the separated aqueous phase is brought to a strongly acidic pH by the addition of one or more of the mineral acids described above.

Acidification of phases b) and d) is advantageously carried out using the same acid, fed at the same or different concentrations.

The dicarboxylic acids present in the aqueous phase are then easily separated from the aqueous environment on the basis of their chemical and physical characteristics, using techniques known to those skilled in the art.

According to a preferred aspect, the aqueous phase after acidification from step d) of the process according to the present invention is cooled to promote precipitation of the dicarboxylic acids and subsequently subjected to solid/liquid separation (filtration).

The dicarboxylic acid thus obtained in solid form may undergo one or more further purification operations to achieve the required degree of purity. Such operations may for example be chosen from extraction, for example with a non-polar solvent, and crystallisation.

For example, further purification of the dicarboxylic acids may follow by adopting, for example, the extraction and crystallisation techniques described in patent application WO2017/211766A1.

In the case of organic solvent extraction, this is preferably performed by sending an aqueous fraction rich in dicarboxylic acids to an extraction column countercurrently to the solvent.

The extraction operations take place at temperatures varying according to the solubility of the dicarboxylic acid being purified; they are typically between 50 and 90°C. In the case of the purification of azelaic acid, the extraction temperatures are preferably between 65 and 90°C at atmospheric pressure.

Organic solvents suitable for use in such extraction are aliphatic and/or aromatic hydrocarbons and/or mixtures thereof and may be chosen from the group consisting of linear or branched, cyclic or acyclic alkanes and aliphatic alkenes such as hexane, cyclohexane, hexene, cyclohexene, methylcyclopentane, methylcyclopentene, 2,2,4-trimethylpentane, methylcyclohexane, heptane, octane, nonane, isooctane; aromatic hydrocarbons such as benzene, toluene, xylene and their analogues, optionally substituted with alkyls having 1 to 6 carbon atoms; mixtures such as petroleum ether and naphtha.

Organic solvents particularly suitable for facilitating the extraction of monocarboxylic acids from the aqueous phase are octane, nonane and their mixtures.

Those skilled in the art will easily be able to modulate the temperature of the extraction operation according to the boiling point of the organic solvent used, the type of dicarboxylic acid to be purified and the possible formation of azeotropes. For the purification of azelaic acid by extraction with octane, for example, temperatures of between 70 and 85°C are advantageous. The dicarboxylic acids present in the aqueous phase are crystallised from solution according to a preferred aspect of the process.

Crystallisation may be carried out in both batch and continuous systems, in crystallisers of any configuration known in the art (for example cooling, evaporation, simple agitation, forced circulation, turbulence, fluidised bed crystallisers).

This crystallisation is preferably carried out in a fractionated manner, that is by subjecting the mixture to be treated to successive stages of evaporation, using at least two crystallisers placed in series and operating at different temperature and pressure conditions, in order to allow optimum separation and purification of the dicarboxylic acid.

Those skilled in the art will easily identify the operating conditions on the basis of the composition of the mixture of dicarboxylic acids to be purified. For example, azelaic acid is advantageously crystallised from an aqueous solution by means of two crystallisers placed in series, operating the first at an absolute pressure of between 200 and 100 mbar, preferably between 180 and 120 mbar, and the second at an even lower absolute pressure depending on the amount of residual azelaic acid in the mother liquors from the first crystallisation, for example at below 50 mbar.

According to one aspect of the process, fractional crystallisation is combined with a solid/liquid separation system according to techniques known to those skilled in the art, for example by centrifuging and/or settling.

According to a preferred aspect, the purified dicarboxylic acids will have a monocarboxylic acid content of less than 0.5 % by weight.

The dicarboxylic acids obtained by the process according to the present invention are aliphatic diacids, preferably of the alpha-omega type, for example chosen from oxalic acid, malonic acid, succinic acid, glutaric acid, adipic acid, pimelic acid, suberic acid, azelaic acid, sebacic acid, undecandicarboxylic acid, dodecandicarboxylic acid, brassylic acid, tetradecandicarboxylic acid, pentadecandicarboxylic acid. Advantageously, said dicarboxylic acids have a C6-C24 chain and preferably belong to the group comprising suberic acid, azelaic acid, sebacic acid, undecandicarboxylic acid, brassylic acid and mixtures thereof.

According to a preferred aspect of the invention, the dicarboxylic acid obtained comprises, or advantageously consists of, azelaic acid.

The dicarboxylic acids obtained by this process are used in the synthesis of polymers such as polyesters, polyamides, polyester-amides, polyurethanes and polyester-urethanes.

### EXAMPLES

### Preparation of the starting mixture

The triglyceride mixture containing starting dicarboxylic acids was obtained from high oleic sunflower oil by the process in patent EP 2 155 646 B1, according to Example 1. In particular, the steam distillation residue (c-phase) of the organic phase obtained by oxidative cleavage of the oil was used.

This distillation residue, analysed by gas chromatography both directly and after derivatisation, was predominantly azelaic acid triglycerides, with an overall azelaic acid content (after trans-methylation with BF₃/MeOH) of 37% by weight of the total weight of the mixture.

Derivatisation was carried out by a trans-methylation reaction with BF₃/MeOH using the following method: BF₃in MeOH (3mL), toluene (1 mL) and 2,2'-dimethoxypropane (150 µL) were added to a known amount of distillation residue (approx. 0.1 g) and internal standard solution.

After incubation in an oven at 80°C for 1 hour, the sample was cooled; 1 mL of solution was withdrawn and transferred into a flask, to which 1 mL of water and 1 mL of chloroform were subsequently added. The solution was shaken and centrifuged at 4000 rpm for 5 minutes. The aqueous phase, comprising the glycerol resulting from the reaction, was separated from the organic phase comprising the methyl esters.

Next, the organic phase was dehydrated with Na2SO4 and injected into the GC-FID.

### Example 1

### Step a)

100 g of distillation residue was placed in an Erlenmeyer flask together with 654 g of 5.7% sodium hydroxide aqueous solution and the resulting mixture was stirred and heated under reflux for 3 hours.

At the end of the saponification reaction, the mixture was cooled to approximately 80°C and transferred to a thermostatted reactor.

### Step b)

A 37% w/w hydrochloric acid solution was added to the saponified product, thermostatted at 80°C, dropwise while stirring until phase separation was achieved (pH 4).

### Step c)

The mixture was then settled for 15 minutes at 80°C to facilitate separation of the aqueous phase rich in azelaic acid from the organic phase.

### Step d)

The aqueous phase was transferred to a container equipped with a stirring and thermostatting system and progressively acidified to pH 1 (with 37% hydrochloric acid by weight) at a temperature of 80°C with stirring.

At a pH of 1 the aqueous phase was cooled to 4°C and kept refrigerated overnight to allow the azelaic acid to precipitate.

The precipitated solid was filtered through a Buchner filter and washed several times by re-dispersing it in cold water until the pH of the wash water was around 5-6.

The washed solid was dried to constant weight (24 g) in an oven and then characterised. GC analysis of a sample of the silanised solid confirmed that azelaic acid had been obtained with a purity of 97.5%, corresponding to a recovery of 63.2% of the total azelaic acid theoretically obtainable from the starting mixture.

### Comparative example 1

Example 1 was replicated by gradually acidifying all the saponification product obtained in step a) (with 37% w/w hydrochloric acid), at a temperature of 80°C and under constant stirring, to pH 1.

At a pH of 1 the reaction product was cooled to 4°C and kept refrigerated overnight to allow the azelaic acid to precipitate.

The precipitated solid was filtered through a Buchner filter and washed several times by re-dispersing it in cold water until the pH of the wash water was around 5-6.

The washed solid was dried to constant weight (12.7g) in an oven and subsequently characterised. GC analysis of a sample of silanised solid confirmed that azelaic acid with a purity of 98.4% had been obtained, corresponding to an azelaic acid recovery of only 33.8% with respect to the amount obtainable from the starting mixture.

## Claims

1. Process for obtaining dicarboxylic acids from a mixture of triglycerides of dicarboxylic acids comprising the steps of:
a) hydrolysing said mixture of triglycerides of dicarboxylic acids in the presence of a basic aqueous solution, obtaining a hydrolysis product emulsion;
b) acidifying the product from step a) until the emulsion separates;
c) separating an aqueous phase containing dicarboxylic acids in a partially dissociated form and glycerol from the product from step b), and
d) acidifying the aqueous phase separated from step c) to a pH of 3 or below, yielding dicarboxylic acids.

2. Process according to claim 1, wherein the hydrolysis in step a) is performed in the presence of a strong base selected from: sodium hydroxide, potassium hydroxide, calcium hydroxide, barium hydroxide, lithium hydroxide and magnesium hydroxide.

3. Process according to any of the claims 1-2, in which said step a) is performed at a temperature between 50°C and 350°C.

4. Process according to claim 3, wherein said step a) is performed at a temperature between 80°C and 150°C.

5. Process according to one or more of claims 1-4, wherein step b) is performed by adding a mineral acid selected from the group consisting of sulfuric acid, hydrochloric acid, nitric acid, phosphoric acid, hydrobromic acid, hydroiodic acid, perchloric acid and the like.

6. Process according to one or more of claims 1-5, wherein the pH reached in step b) has a value of 3.5 to 7.

7. Process according to one or more of claims 1-6, wherein step c) comprises at least one operation selected from centrifuging, settling, solvent extraction and combinations thereof.

8. Process according to one or more of claims 1-7, wherein said mixture of triglycerides of dicarboxylic acids is obtained as a product of a process of the oxidative cleavage of vegetable oils (or mixtures of vegetable oils) containing unsaturated carboxylic acids.

9. Process according to one or more of claims 1-8 comprising, before to step a), the preliminary steps of:
(i) hydrolysing said triglyceride mixture of dicarboxylic acids in the absence of a base,
(ii) separating a first aqueous phase, comprising glycerol, from a first organic phase containing the remaining hydrolysis product from step (i), and
(iii) evaporating and/or distilling said first organic phase to remove the free dicarboxylic and monocarboxylic acids contained therein,
obtaining an evaporation and/or distillation residue comprising a mixture of unhydrolysed or partially hydrolysed glycerides of dicarboxylic acids to be fed to step a).

10. Process according to one or more of claims 1-9, wherein the dicarboxylic acids obtained in step d) are crystallised from the aqueous solution.

## Patentansprüche

1. Verfahren zur Gewinnung von Dicarbonsäuren aus einer Mischung von Triglyceriden von Dicarbonsäuren, umfassend die Schritte:
a) das Hydrolysieren der Mischung von Triglyceriden von Dicarbonsäuren in Gegenwart einer basischen wässrigen Lösung, wobei eine Hydrolyseproduktemulsion erhalten wird;
b) das Ansäuern des Produkts aus Schritt a), bis sich die Emulsion trennt;
c) das Abtrennen einer wässrigen Phase, enthaltend Dicarbonsäuren in teilweise dissoziierter Form und Glycerin, von dem Produkt aus Schritt b), und
d) das Ansäuern der in Schritt c) abgetrennten wässrigen Phase auf einen pH-Wert von 3 oder niedriger, wodurch Dicarbonsäuren erhalten werden.

2. Verfahren gemäß Anspruch 1, wobei die Hydrolyse in Schritt a) in Gegenwart einer starken Base durchgeführt wird, die aus Natriumhydroxid, Kaliumhydroxid, Calciumhydroxid, Bariumhydroxid, Lithiumhydroxid und Magnesiumhydroxid ausgewählt ist.

3. Verfahren gemäß einem der Ansprüche 1-2, wobei Schritt a) bei einer Temperatur zwischen 50°C und 350°C durchgeführt wird.

4. Verfahren gemäß Anspruch 3, wobei Schritt a) bei einer Temperatur zwischen 80°C und 150°C durchgeführt wird.

5. Verfahren gemäß einem oder mehreren der Ansprüche 1-4, wobei Schritt b) durch Hinzufügen einer Mineralsäure durchgeführt wird, die aus der Gruppe ausgewählt ist, die aus Schwefelsäure, Salzsäure, Salpetersäure, Phosphorsäure, Bromwasserstoffsäure, Iodwasserstoffsäure, Perchlorsäure und dergleichen besteht.

6. Verfahren gemäß einem oder mehreren der Ansprüche 1-5, wobei der in Schritt b) erreichte pH-Wert einen Wert von 3,5 bis 7 aufweist.

7. Verfahren gemäß einem oder mehreren der Ansprüche 1-6, wobei Schritt c) mindestens einen Vorgang umfasst, der aus Zentrifugieren, Absetzen, Lösungsmittelextraktion und Kombinationen davon ausgewählt ist.

8. Verfahren gemäß einem oder mehreren der Ansprüche 1-7, wobei die Mischung von Triglyceriden von Dicarbonsäuren als Produkt eines Verfahrens des oxidativen Spaltens von Pflanzenölen (oder Mischungen von Pflanzenölen), die ungesättigte Carbonsäuren enthalten, erhalten wird.

9. Verfahren gemäß einem oder mehreren der Ansprüche 1-8, umfassend vor Schritt a) die vorbereitenden Schritte:
(i) das Hydrolysieren der Triglyceridmischung von Dicarbonsäuren in Abwesenheit einer Base,
(ii) das Abtrennen einer ersten wässrigen Phase, die Glycerin umfasst, von einer ersten organischen Phase, die das verbleibende Hydrolyseprodukt aus Schritt (i) enthält, und
(iii) das Verdampfen und/oder Destillieren der ersten organischen Phase, um die darin enthaltenen freien Dicarbonsäuren und Monocarbonsäuren zu entfernen,
das Erhalten eines Verdampfungs- und/oder Destillationsrückstand, der eine Mischung aus nicht-hydrolysierten oder teilweise hydrolysierten Glyceriden von Dicarbonsäuren umfasst, die in Schritt a) zugeführt wird.

10. Verfahren gemäß einem oder mehreren der Ansprüche 1-9, wobei die in Schritt d) erhaltenen Dicarbonsäuren aus der wässrigen Lösung kristallisiert werden.

## Revendications

1. Procédé d'obtention d'acides dicarboxyliques à partir d'un mélange de triglycérides d'acides dicarboxyliques comprenant les étapes de :
a) hydrolyse dudit mélange de triglycérides d'acides dicarboxyliques en présence d'une solution aqueuse basique, permettant l'obtention d'une émulsion de produit d'hydrolyse ;
b) acidification du produit de l'étape a) jusqu'à ce que l'émulsion se sépare ;
c) séparation d'une phase aqueuse contenant des acides dicarboxyliques sous une forme partiellement dissociée et du glycérol du produit de l'étape b), et
d) acidification de la phase aqueuse séparée de l'étape c) à un pH inférieur ou égal à 3, produisant des acides dicarboxyliques.

2. Procédé selon la revendication 1, dans lequel l'hydrolyse à l'étape a) est réalisée en présence d'une base forte choisie parmi : l'hydroxyde de sodium, l'hydroxyde de potassium, l'hydroxyde de calcium, l'hydroxyde de baryum, l'hydroxyde de lithium et l'hydroxyde de magnésium.

3. Procédé selon l'une quelconque des revendications 1-2, dans lequel ladite étape a) est réalisée à une température comprise entre 50 °C et 350 °C.

4. Procédé selon la revendication 3, dans lequel ladite étape a) est réalisée à une température comprise entre 80 °C et 150 °C.

5. Procédé selon une ou plusieurs des revendications 1-4, dans lequel l'étape b) est réalisée en ajoutant un acide minéral choisi dans le groupe consistant en l'acide sulfurique, l'acide chlorhydrique, l'acide nitrique, l'acide phosphorique, l'acide bromhydrique, l'acide iodhydrique, l'acide perchlorique et analogues.

6. Procédé selon une ou plusieurs des revendications 1-5, dans lequel le pH atteint à l'étape b) présente une valeur de 3,5 à 7.

7. Procédé selon une ou plusieurs des revendications 1-6, dans lequel l'étape c) comprend au moins une opération choisie parmi la centrifugation, la décantation, l'extraction par solvant et des combinaisons de celles-ci.

8. Procédé selon une ou plusieurs des revendications 1-7, dans lequel ledit mélange de triglycérides d'acides dicarboxyliques est obtenu en tant que produit d'un procédé de clivage oxydatif d'huiles végétales (ou de mélanges d'huiles végétales) contenant des acides carboxyliques insaturés.

9. Procédé selon une ou plusieurs des revendications 1-8 comprenant, avant l'étape a), les étapes préliminaires de :
(i) hydrolyse dudit mélange de triglycérides d'acides dicarboxyliques en l'absence d'une base,
(ii) séparation d'une première phase aqueuse, comprenant du glycérol, d'une première phase organique contenant le produit d'hydrolyse restant de l'étape (i), et
(iii) évaporation et/ou distillation de ladite première phase organique pour éliminer les acides dicarboxyliques et monocarboxyliques libres contenus dans celle-ci,
permettant l'obtention d'un résidu d'évaporation et/ou de distillation comprenant un mélange de glycérides non hydrolysés ou partiellement hydrolysés d'acides dicarboxyliques à introduire dans l'étape a).

10. Procédé selon une ou plusieurs des revendications 1-9, dans lequel les acides dicarboxyliques obtenus à l'étape d) sont cristallisés à partir de la solution aqueuse.
